(19) Europäisches Patentamt
European Patent Office
Office européen des brevets

(11) Publication number: 0 106 486
A2

(12) # EUROPEAN PATENT APPLICATION

(21) Application number: 83305132.9

(22) Date of filing: 05.09.83

(51) Int. Cl.³: C 07 D 221/10
C 07 D 407/06, C 07 D 409/06
A 61 K 31/47
//C07C43/23, C07C43/215

(30) Priority: 07.09.82 US 415498

(43) Date of publication of application:
25.04.84 Bulletin 84/17

(84) Designated Contracting States:
AT BE CH DE FR GB IT LI LU NL SE

(71) Applicant: ELI LILLY AND COMPANY
307, East McCarty Street
Indianapolis Indiana 46285(US)

(72) Inventor: Zimmerman, Dennis Michael
121 Thorncrest Drive
Mooresville Indiana 46158(US)

(74) Representative: Crowther, Terence Roger et al,
Erl Wood Manor
Windlesham Surrey GU20 6PH(GB)

(54) **Improvements in or relating to novel octahydrobenz(f)isoquinolines.**

(57) It has been found that novel octahydrobenz[f]isoquinoline derivatives, or a pharmaceutically-acceptable salt thereof, are useful analgesic agents, having both narcotic agonist and antagonist properties, as well as psychotropic properties.

EP 0 106 486 A2

# IMPROVEMENTS IN OR RELATING TO NOVEL OCTAHYDROBENZ[f]ISOQUINOLINES

It has long been known that slight chemical modifications of the morphine molecule lead to analgesic agonists of widely differing potencies and addictive properties. For example, codeine, the methyl ether of morphine, is a relatively mild analgesic agonist having only slight dependance (addiction) probability. On the other hand, heroin, the diacetyl derivative of morphine, is a powerful agonist with extremely high addiction potential. In addition, as long ago as 1915, Pohl found that when the N-methyl group of codeine was replaced with an allyl group, the resulting compound, N-allylnorcodeine, was an opiate antagonist. In 1940, N-allylnormorphine or nalorphine was synthesized and was shown to have a highly specific ability to reverse the depressant effects of morphine. Other simple chemical modifications of the morphine molecule have yielded many interesting drugs. Thus, one fruitful research area in the search for improved analgesics of high potency or lower addiction liability has been the chemical modification of the morphine molecule.

In addition to chemically modifying the morphine ring structure, chemists have developed a second related field of research--the preparation of compounds containing only part-structures of morphine--with the same end in mind as above; i.e., the synthesis of improved analgesic agonists and/or analgesic antagonists of improved properties. For example, meperidine,

a widely used analgesic, can be written as a morphine part-structure, as can the benzomorphans and the prodines. Many other compounds of morphine part-structures have been prepared, some of which have improved analgesic agonist properties and others, particularly those with an allyl group attached to a ring nitrogen, which have opiate antagonist properties. It had been hoped that morphine part-structure research would produce a compound having both opiate agonist and antagonist properties since the opiate antagonist property would assure a user that the compound would have a greatly reduced dependance liability. Two recently marketed analgesics, pentazocine and phenazocine, have been found to be both antagonists and agonists although they still retain a certain degree of opiate dependance liability.

Recently, two groups have prepared isoquinoline analogs of the prodines and/or the benzomorphans. Menard, *et al.*, *Can. J. Chem.*, *52*, 2316 (1974), prepared several octahydrobenz[f]isoquinolines which were reported to possess analgesic activity. All of the compounds prepared and tested were substituted at the 10b position with hydroxy or acyloxy.

Hahne, *et al.*, *Arch. Pharm.*, *312*, 472 (1979), reported the preparation and analgesic activity of octahydrobenz[f]isoquinolines which are not substituted in the benzene portion of the molecule.

In accordance with the present invention, novel substituted octahydrobenz[f]isoquinoline derivatives are useful as analgesic agents, having both narcotic agonist and antagonist properties, as well as psychotropic characteristics.

Accordingly, compounds of formula (I)

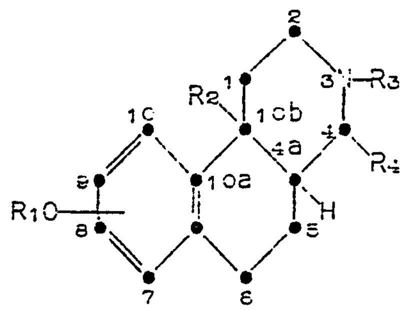

I

or a pharmaceutically-acceptable salt thereof, in which

$R_1$ is hydrogen, $C_1$-$C_4$ alkyl, or $C_1$-$C_{12}$ alkanoyl;

$R_2$ is $C_1$-$C_4$ alkyl, $C_2$-$C_6$ alkenyl, or

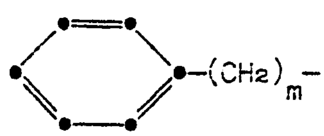

in which m is 1, 2, or 3;

$R_3$ is hydrogen, $C_1$-$C_8$ alkyl, $C_2$-$C_6$ alkenyl-methyl, ($C_3$-$C_8$ cycloalkyl)methyl,

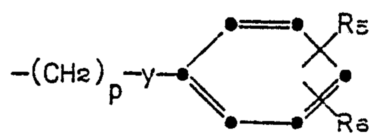

, or

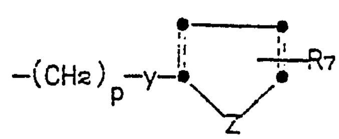

,

in which p is 0, 1, or 2;

Y is $-\overset{O}{\overset{\|}{C}}-$, $-\overset{OH}{\overset{|}{C}H}-$, $-CH_2-$, -O-, -S-, or -NH-, providing that when Y is -O-, -S-, or -NH-, p may only be 2, and providing that when Y is $-\overset{OH}{\overset{|}{C}H}-$ , p may not be 0;

Z is O or S;

$R_5$ is $C_1$-$C_4$ alkylthio, nitro, amino, trifluoromethyl, hydroxy, hydrogen, $C_1$-$C_4$ alkyl, $C_1$-$C_4$ alkoxy, or halo;

$R_6$ is hydrogen, $C_1$-$C_4$ alkyl, $C_1$-$C_4$ alkoxy, or halo;

$R_7$ is hydrogen or methyl; and

$R_4$ is $C_1$-$C_8$ alkyl or hydrogen, are useful analgesic and psychotropic agents.

In a further aspect of the invention, there is provided a process for preparing a compound of Formula (I), or a pharmaceutically-acceptable salt thereof, said process comprising

(a) Reducing a compound of Formula (II),

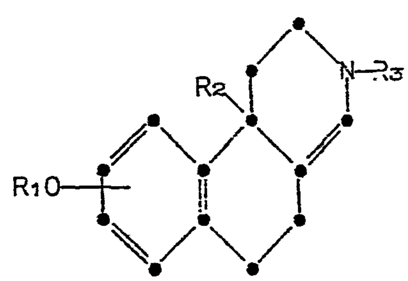

(II)

to form a compound of Formula (I) in which $R_4$ is hydrogen; or,

(b) Reacting a compound of Formula (III),

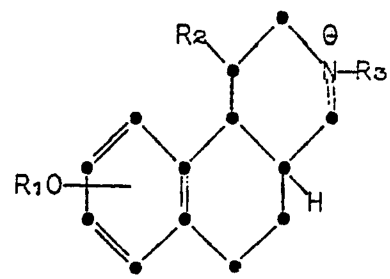

(III)

in which $R_1$ is $C_1$-$C_4$ alkyl, with an organometallic reagent to form a compound of Formula (I), in which $R_4$ is $C_1$-$C_8$ alkyl; or,

(c) Reducing a compound of Formula III to form a compound of Formula I in which $R_4$ is hydrogen; or

(d) Hydrolysis or reduction of a compound of Formula (I) in which $R_1$ is $C_1$-$C_4$ alkyl or $C_1$-$C_{12}$ alkanoyl, to form a compound in which $R_1$ is hydrogen; or,

(e) Acylation or alkylation of a compound of Formula (I) in which $R_1$ is H, to form a compound of Formula (I) in which $R_1$ is alkanoyl or alkyl ; or

(f) Treatment of a compound of Formula (I), in which $R_3$ is $-\underset{\underset{O}{\|}}{C}-OW$ where W is $C_1$-$C_4$ alkyl, benzyl or phenyl, with a base to form a compound of Formula (I) in which $R_3$ is hydrogen; or,

(g) Reacting a compound of Formula (I) in which $R_3$ is hydrogen, with an alkyl halide, or acyl halide followed by reduction, to form a compound of Formula (I) in which $R_3$ is other than hydrogen; and,

(h) if desired, salifying a product of any of reactions (a)-(g).

The term "$C_1$-$C_8$ alkyl" refers to straight and branched alkyl radicals of one to eight carbon atoms and includes methyl, ethyl, n-propyl, isopropyl, tert-butyl, hexyl, octyl, and includes the terms "$C_1$-$C_4$ alkyl" and "$C_2$-$C_4$ alkyl". The term "$C_2$-$C_6$ alkenyl" refers to straight and branched alkene radicals of two to six carbon atoms such as vinyl, allyl, 4-methyl-3-pentenyl, or 5-hexenyl. The term "$C_2$-$C_6$ alkenylmethyl" refers to straight and branched alkene radicals of two to six carbon atoms attached to a methyl radical, such as 2-propenyl (allyl), 2-butenyl, 3-butenyl, or 2-pentenyl. The term "($C_3$-$C_8$ cycloalkyl)methyl" refers to a methyl radical substituted with saturated alicyclic rings of three to eight carbon atoms, such as cyclopropylmethyl, methylcyclopropylmethyl, cyclobutylmethyl, cyclopentylmethyl, cyclohexylmethyl, 1-, 2-, 3-, or 4-methylcyclohexylmethyl, or cycloheptylmethyl, cyclooctylmethyl.

The term "$C_1$-$C_4$ alkoxy" refers to the straight and branched aliphatic ether radicals of one to four carbon atoms such as methoxy, ethoxy, propoxy, isopropoxy, butoxy, isobutoxy, sec-butoxy, or tert-butoxy.

The term "$C_1$-$C_{12}$ alkanoyl" refers to straight and branched aliphatic acyl radicals of one to twelve carbon atoms, such as formyl, acetyl, propionyl, pentanoyl, 4-butyloctanoyl, or lauroyl.

The term "halo" refers to fluoro, chloro, bromo, and iodo.

The pharmaceutically-acceptable salts, including quaternary ammonium salts, of the amine bases represented by the above formula are formed with acids, and include, for example, salts derived from inorganic acids such as hydrochloric acid, nitric acid, phosphoric acid, sulfuric acid, hydrobromic acid, hydroiodic acid, nitrous acid, or phosphorous acid, as well as salts derived from organic acids including aliphatic mono and dicarboxylic acids, phenyl-substituted alkanoic acids, hydroxy alkanoic and alkanedioic acids, aromatic acids, aliphatic and aromatic sulfonic acids. Such pharmaceutically-acceptable salts may include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, or naphthalene-2-sulfonate. "Pharmaceutically-acceptable salts" are those salts useful in the chemotherapy of warm-blooded animals.

The usual nonreactive solvents used in the preparation of the salt from the base include acetone, tetrahydrofuran, diethyl ether, or ethyl acetate. Quaternary salts can be prepared in generally the same way by reaction of the base with an alkyl sulfate or alkyl halide, for example, methyl sulfate, methyl iodide, ethyl bromide, or propyl iodide.

The inorganic acid salts, especially the hydrochloride salts, are preferred.

The preferred compounds of this invention are those in which

a) $R_1$ is $C_1$-$C_4$ alkyl, especially methyl,

b) $R_1$ is hydrogen,

c) $R_2$ is methyl,

d) $R_2$ is n-propyl,

e) $R_3$ is hydrogen,

f) $R_3$ is $C_1$-$C_4$ alkyl, especially methyl,

g) $R_3$ is $C_2$-$C_6$ alkenylmethyl, especially 2-propenyl (allyl),

h) $R_3$ is cyclopropylmethyl,

i) $R_3$ is $-(CH_2)_p-Y-$ 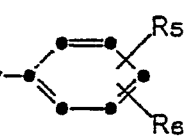 , especially 2-phenylethyl, and

j) $R_4$ is hydrogen.

Especially preferred are those compounds wherein $R_1O$ is at the 9-position of the molecule.

The bridgehead substituents, the hydrogen at 4a and $R_2$ at 10b can have either a cis or trans relationship to one another; i.e., the two substituents can be on the same "side" of the octahydroisoquinoline ring system (cis) or on opposite "sides" (trans). In addition, both the 4a and 10b carbon atoms are asymmetric, thus giving rise in each compound to 4 optical isomers, occurring as two optical isomer mixtures designated as the cis-dl and the trans-dl pair. Formula I is thus intended to comprehend both the optical isomers, the cis-dl and trans-dl mixtures, and their individual enantiomorphs since, as far as is known, all of the individual isomers and isomer mixtures are useful as psychotropic agents and as analgesic agonists or as analgesic antagonists, albeit large quantitative differences in agonist or antagonistic potency may exist between individual isomers or racemates. Those compounds of Formula I which are in the cis configuration, however, are preferred; i.e., the cis-dl pair and the individual cis isomers such as the cis-l and cis-d compounds. Where $R_4$ is other than hydrogen, an additional chiral center is present; both possible isomers and their mixture are contemplated by this invention.

The novel compounds of this invention are prepared according to scheme I:

Scheme I

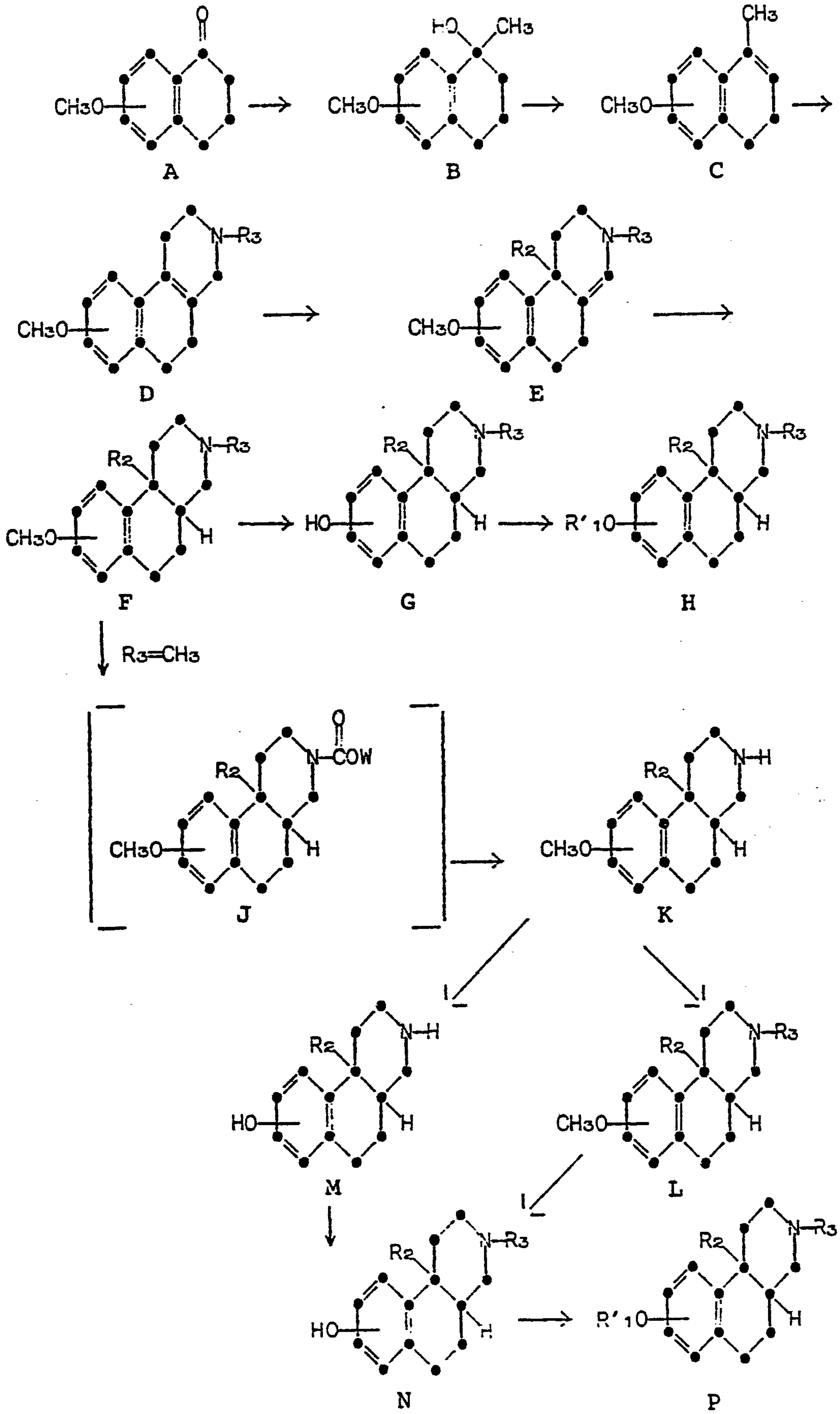


A B C

D E

F G H

J K

M L

N P

In scheme I, $R_2$ and $R_3$ are the same as described earlier, $R'_1$ is $C_2$-$C_4$ alkyl or $C_1$-$C_{12}$ alkanoyl, and W is $C_1$-$C_4$ alkyl, benzyl, or phenyl.

A methoxy-1-tetralone (A) is converted to the corresponding methoxy-1-hydroxy-1-methyl-1,2,3,4-tetrahydronaphthalene (B) by treatment with a methyl Grignard reagent in the usual manner. Treatment of the hydroxy intermediate (B) with a dehydrating agent, such as polyphosphoric acid, sulfuric acid, or p-toluenesulfonic acid in a solvent such as benzene, xylene, toluene, or the like with the concurrent removal of water, usually at elevated temperatures, results in the loss of water and the formation of a methoxy-1-methyl-3,4-dihydronaphthalene (C). Aminomethylation of the latter intermediate with two equivalents of formaldehyde, and the appropriate $R_3$-amine gives a methoxy-3-$R_3$-substituted-1,2,3,4,5,6-hexahydrobenz[f]isoquinoline (D) which is the required intermediate for all subsequent transformations.

Treatment of the methoxy-3-$R_3$-substituted-1,2,3,4,5,6-hexahydrobenz[f]isoquinoline (D) with a strong base, such as n-butyl lithium, sodium amide, or methyl lithium, in a compatible solvent, such as ethers, tetrahydrofuran, alkanes, such as hexane, and the like, preferably at low temperatures, for instance 0°C. to -78°C., followed by the addition of the resulting anion to a solution of the desired $R_2$-halide, results in the preparation of the corresponding methoxy-10b-$R_2$-substituted-3-$R_3$-substituted-1,2,3,5,6,10b-hexahydrobenz[f]isoquinoline (E). Suitable $R_2$ groups are

those as defined earlier, and suitable halide groups may include chloro, bromo, and iodo. Thus, suitable $R_2$-halides for the above reaction may include methyl bromide, n-butyl iodide, isopropyl chloride, ethyl bromide, 5-hexenyl bromide, or methyl iodide.

Reduction of the hexahydro derivative above to the corresponding methoxy-10b-$R_2$-substituted-3-$R_3$-substituted-9-methoxy-1,2,3,4,4a,5,6,10b-octahydrobenz[f]isoquinoline (F) may be accomplished by any of a number of methods well known to those skilled in the art. More specifically, the reduction may be accomplished by reaction with hydride reagents such as sodium borohydride, diborane, lithium aluminum hydride, or sodium bis-(2-methoxyethoxy)aluminum hydride, either on the compound in its free base form, or on a suitable salt, such as the perchlorate salt. The hydride reduction reactions are generally carried out in the presence of a suitable solvent, for example ethers such as diethyl ether, dipropyl ether, tetrahydrofuran, diglyme, or in alcohols such as methanol, ethanol, or isopropanol. Weak acids, such as formic acid or acetic acid may be added to the reaction mixture if desired, thereby making the reduction more efficient. A preferred reduction method, for example, comprises treating the hexahydro derivative with sodium borohydride in the presence of a suitable solvent, such as tetrahydrofuran, and in the presence of an acid such as acetic acid. This reaction gives predominantly the preferred cis compounds of this invention. The amount of reducing agent used can vary over a wide range.

Generally, the amount of reducing agent used is in excess of the hexahydro derivative, normally from about 1 to about 10 molar excess; however, more or less can be used if desired. The reaction is usually carried out at a temperature below about 180°C., although the precise temperature is not critical. Preferably, the reactants are mixed while the temperature is maintained at about 0° to about 20° C. After the reactants have been combined, the temperature can be increased, preferably to a temperature from about 50° to about 150°C. Normally, the reduction is complete within about 1 to about 4 hours. The product can be recovered by making the reaction mixture alkaline, for example by adding an aqueous base such as sodium hydroxide, potassium hydroxide, or ammonium hydroxide and extracting the aqueous alkaline reaction mixture with a water immiscible organic solvent, such as diethyl ether, ethyl acetate, or dichloromethane.

The reduction of the hexahydro compound (E) can be accomplished alternatively by hydrogenation. The hydrogenation is carried out preferably in an unreactive solvent such as tetrahydrofuran, dioxane, ethanol, methanol, water, or N,N-dimethylformamide, under neutral, acidic, or basic conditions. The particular solvent used is not critical, but preferably the solvent selected is one in which the hexahydro derivative will be at least partially soluble. The hydrogenation is carried out in the presence of a suitable catalyst such as Raney nickel, platinum oxide, platinum, or palladium on a suitable carrier such as

carbon or barium sulfate. The particular reaction conditions are not critical to the process, but generally the hydrogenation is carried out at a temperature of about 25° to about 200°C., with a hydrogen gas pressure of about 20 to about 100 p.s.i. The particular catalyst used in the reaction can determine to some degree the particular octahydro isomer obtained. When the hydrogenation is carried out in the presence of palladium on carbon, for example, the octahydro compound is predominantly the cis isomer. Mixtures of cis and trans compounds can be separated, if desired, by any of the commonly used techniques such as distillation, gas liquid chromatography, high pressure liquid chromatography, solid liquid chromatography, or crystallization. In this manner, the compounds in which $R_1$ and $R_3$ are both methyl are prepared. These compounds are also useful as intermediates for preparing the other compounds of this invention.

For instance, compounds of structure G are prepared by the cleavage of the methoxy group to the corresponding phenol using known methods such as hydrobromic acid and acetic acid, or boron tribromide, for example. Such methods of ether cleavage are well known to those in the art; see for example U.S. Patent No. 3,324,139. The phenol compounds are useful as analgesic agents and additionally serve as intermediates to $C_1$-$C_4$-alkylether derivatives. These phenols may be alkylated with $C_1$-$C_4$ alkyl halides in which $C_1$-$C_4$ alkyl and halide are as defined earlier optionally in the presence of an acid binding agent, such as sodium

hydroxide, potassium carbonate, triethylamine, or magnesium hydroxide, to give the appropriate $R'_1$-substituted-3-methyl-10b-$R_2$-substituted product (H). Typically, equimolar quantities of the phenol and the alkylating agent are stirred together, preferably in a non-reactive solvent, including amides such as dimethyl formamide or dimethylacetamide, or sulfoxides such as dimethyl sulfoxide at temperatures from about 0°C. to the reflux temperature of the solution, preferably at about ambient temperature. The alkanoyl derivatives are similarly prepared by the action of an appropriate $C_1$-$C_{12}$ alkanoyl halide on the phenol in like manner to also provide compounds of structure H.

Alternatively, the appropriate methoxy-3-methyl-10b-$R_2$-substituted-1,2,3,4,4a,5,6,10b-octahydrobenz[f]isoquinolines (F, $R_3$ is methyl) may be demethylated to the corresponding 3-hydrogen compound (K) by treating compound F with a haloformate to provide a carbamate (J, W is $C_1$-$C_4$ alkyl, benzyl, or phenyl) which gives a secondary amine (K) when treated with a base. This type of reaction is described in more detail by Abdel-Monen and Portoghese, J. Med. Chem., 15, 208 (1972). In particular a haloformate such as, for example, phenyl chloroformate, ethyl bromoformate, or benzyl chloroformate is reacted with a tertiary amine, for example a 3-methyl isoquinoline derivative in the present case, to afford a carbamate. The reaction is generally carried out in an unreactive solvent such as dichloromethane, chloroform, acetone, or ethyl acetate. The temperature is usually held

below about 200° C., and the reaction is substantially complete within about 1 to 5 hours. The product carbamate (J) can be isolated by simply evaporating the reaction solvent, and further purification of the product is generally not needed. The carbamate (J) is converted to the 3-hydrogen compound by the action of a suitable base such as aqueous sodium hydroxide or aqueous potassium carbonate. A suitable organic solvent is normally added to the reaction mixture in order to dissolve the carbamate. Suitable solvents may include alcohols such as ethanol or methanol, or ethers such as dioxane or tetrahydrofuran. The hydrolysis reaction is generally complete within about 12 to 36 hours when carried out at a temperature of about 50° to about 150°C. The product may be isolated by extracting the aqueous reaction mixture with a suitable solvent such as diethyl ether or methylene chloride. The unsubstituted compound (K) can be further purified by methods such as chromatography, crystallization, or distillation.

These 3-hydrogen compounds (K) are useful analgesic agents and also may be transformed into the corresponding methoxy 3-$R_3$-substituted-10b-$R_2$-substituted-1,2,3,4,4a,5,6,10b-octahydrobenz[f]isoquinolines (L) in the usual manner, or the methoxy functionality may be cleaved to the phenol (M) using hydrobromic acid/acetic acid. The phenol (M) may then be alkylated at the 3-position with a $R_3$-halide as before to yield (N) and subsequently alkylated at the phenol position with a $R'_1$-halide to give (P) if desired.

The alkylation of a phenol derivative ($R_1$ is hydrogen) and most of the 3-hydrogen derivatives ($R_3$ is hydrogen) may be performed in the usual manner with the appropriate alkyl halide reagents in the presence of an acid binding agent. The reactants are gènerally mixed in about equimolar quantities, preferably in an organic solvent. Typical solvents commonly used include amides such as dimethylformamide or dimethylacetamide or sulfoxides such as dimethyl sulfoxide. Generally, a base is added to the reaction mixture to act as an acid binding agent. Typical bases include sodium bicarbonate, potassium carbonate, magnesium hydroxide, or triethylamine. The alkylation is generally complete after about 1 to about 16 hours when carried out at a temperature of about 20° to 120°C. The products are isolated by general methods and further purified if desired. If both $R_1$ and $R_3$ are hydrogen, the 3-position will be selectively alkylated with one equivalent of halide, although alkylation at the phenol position will occur if more than one equivalent of halide is employed.

The introduction of certain $R_3$ substitutents may additionally be accomplished by treating the 3-hydrogen derivative with an appropriate acyl halide, followed by reduction of the resulting carbamate. This process may be used when there is to be a methylene group adjacent to the nitrogen atom. For instance, ($C_3$-$C_8$ cycloalkyl)methyl compounds may be formed by using a $C_3$-$C_8$ cycloalkyl carboxylic acid halide, followed by reduction. The 2-phenylethyl functionality may be introduced by using a phenylacetyl halide,

followed by reduction. Typically, the carboxylic acid chloride is used, usually in the presence of an acid scavenger, such as triethylamine or sodium bicarbonate, in a compatible solvent, such as benzene, dimethylformamide, or dioxane. The carbamate intermediate may be isolated and reduced to the methylene compound with, for instance, lithium aluminum hydride in tetrahydrofuran. In a similar manner, the $R_3$ substituents in which Y is $-\overset{O}{\overset{\|}{C}}-$ may be transformed into the corresponding alcohol and methylene compounds by partial or total reduction of the ketone.

In Scheme I, all four methoxy tetralones (A) are commercially available and thus give the corresponding methoxy compounds of this invention (F). This provides, through cleavage to the corresponding phenol and subsequent alkylation or acylation, an efficient synthesis to $R'_1$-substituted compounds of this invention. As will be obvious to those skilled in the art, other alkoxy tetralones may be used as starting materials in this sequence to give the corresponding compounds of this invention directly. The hydroxy or acyloxy congeners of (A) cannot be utilized as starting materials because side reactions will occur.

Scheme I describes the preparation of those preferred compounds of this invention in which $R_4$ is hydrogen. In the preferred sodium borohydride reduction of the compounds of Structure E, the salt form of E, especially the perchlorate salt, is usually employed. This provides for the following Scheme II:

Scheme II

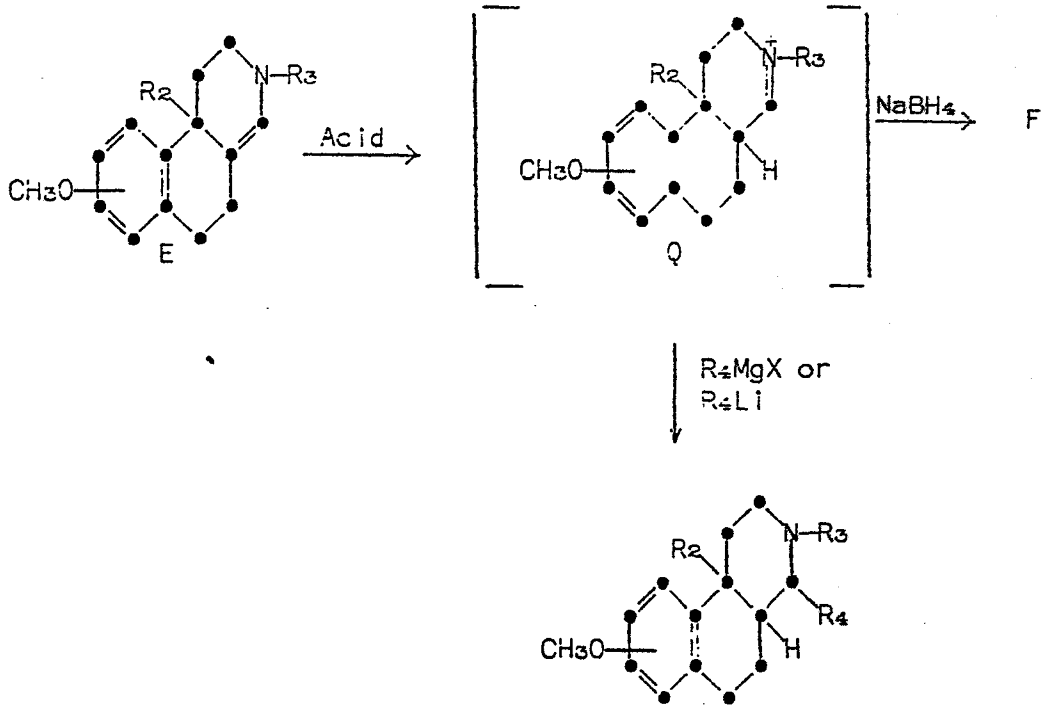


S

Treatment of E with acid shifts the double bond to the iminium compound Q which on treatment with sodium borohydride gives F as previously described. Alternatively, Q may be reacted with a Grignard reagent ($R_4MgX$) or lithium reagent ($R_4Li$) in which $R_4$ and X are the same as previously defined, in the usual manner, to give the 4-$R_4$-substituted compounds of this invention (S). These compounds may be manipulated (i.e., demethylation, ether cleavage, alkylation, acylation) in the same manner as presented in Scheme I to provide the other $R_4$-substituted compounds of this invention.

Typical octahydrobenz[f]isoquinolines provided by this invention include the following:

1,2,3,4,4a,5,6,10b-octahydro-3-isopropyl-9-isopropoxy-10b-methylbenz[f]isoquinoline,

1,2,3,4,4a,5,6,10b-octahydro-3,4-dimethyl-10b-propylbenz[f]isoquinolin-9-ol,

1,2,3,4,4a,5,6,10b-octahydro-3-cyclohexylmethyl-8-n-butoxy-10b-ethylbenz[f]isoquinoline,

1,2,3,4,4a,5,6,10b-octahydro-3-(2-phenoxyethyl)-10b-methylbenz[f]isoquinolin-7-ol,

1,2,3,4,4a,5,6,10b-octahydro-9-methoxy-10b-n-butylbenz[f]isoquinoline hydrobromide,

1,2,3,4,4a,5,6,10b-octahydro-4-butyl-9-acetoxy-10b-ethylbenz[f]isoquinoline,

1,2,3,4,4a,5,6,10b-octahydro-3-(2-butenyl)-10b-isopropylbenz[f]isoquinolin-10-ol,

1,2,3,4,4a,5,6,10b-octahydro-4-isopropyl-9-ethoxy-10b-(4-methyl-3-pentenyl)benz[f]isoquinoline,

1,2,3,4,4a,5,6,10b-octahydro-3-cyclopropylmethyl-8-sec-butoxy-10b-ethylbenz[f]isoquinoline sulfate,

1,2,3,4,4a,5,6,10b-octahydro-10-ethoxy-10b-n-propylbenz[f]isoquinoline,

1,2,3,4,4a,5,6,10b-octahydro-3-cycloheptylmethyl-7-n-propoxy-10b-n-butylbenz[f]isoquinoline,

1,2,3,4,4a,5,6,10b-octahydro-3-(3-butenyl)-10b-sec-butylbenz[f]isoquinolin-9-ol,

1,2,3,4,4a,5,6,10b-octahydro-3-hexyl-4-methyl-9-methoxy-10b-ethylbenz[f]isoquinoline hydrochloride,

1,2,3,4,4a,5,6,10b-octahydro-3-(4-methylcyclohexylmethyl)-10-ethoxy-10b-t-butylbenz[f]isoquinoline,

1,2,3,4,4a,5,6,10b-octahydro-3-[3-(3,4-dichlorophenyl)propyl]-9-methoxy-10b-propylbenz[f]-isoquinoline,

1,2,3,4,4a,5,6,10b-octahydro-10b-benzylbenz[f]isoquinolin-8-ol,

1,2,3,4,4a,5,6,10b-octahydro-10-acetoxy-10b-methylbenz[f]isoquinoline,

1,2,3,4,4a,5,6,10b-octahydro-10b-(3-phenylpropyl)benz[f]isoquinolin-9-ol,

1,2,3,4,4a,5,6,10b-octahydro-3-[2-(2,4-dihydroxyphenyl)ethyl]-10b-(3-phenylpropyl)benz[f]-isoquinolin-7-ol,

1,2,3,4,4a,5,6,10b-octahydro-3-(4-trifluoromethylbenzyl)-8-ethoxy-10b-methylbenz[f]isoquinoline,

1,2,3,4,4a,5,6,10b-octahydro-3-[2-(5-methyl-2-furyl)ethyl]-10b-hexylbenz[f]isoquinolin-9-ol,

1,2,3,4,4a,5,6,10b-octahydro-3-methyl-4-ethyl-10b-hexylbenz[f]isoquinolin-8-ol,

1,2,3,4,4a,5,6,10b-octahydro-3-benzyl-10-acetoxy-10b-octylbenz[f]isoquinoline,

1,2,3,4,4a,5,6,10b-octahydro-3-[2-(2-furylmercapto)ethyl]-7-propionyloxy-10b-ethylbenz[f]isoquinoline,

1,2,3,4,4a,5,6,10b-octahydro-4-methyl-9-methoxy-10b-(2-phenylethyl)benz[f]isoquinoline,

1,2,3,4,4a,5,6,10b-octahydro-3-[3-(3-methoxy-4-chlorophenyl)propyl]-8-formoyl-10b-(5-hexenyl)benz-[f]isoquinoline,

1,2,3,4,4a,5,6,10b-octahydro-3-[2-(4-chloroanilino)-ethyl]-4,10b-dimethylbenz[f]isoquinolin-7-ol,

1,2,3,4,4a,5,6,10b-octahydro-3-[2-(2-thienyl)-ethyl]-4-ethyl-7-methoxy-10b-methylbenz[f]isoquinoline,

1,2,3,4,4a,5,6,10b-octahydro-3-(3-methylbenzyl)-10b-methylbenz[f]isoquinolin-9-ol,

1,2,3,4,4a,5,6,10b-octahydro-4-methyl-8-methoxy-10b-allylbenz[f]isoquinoline,

1,2,3,4,4a,5,6,10b-octahydro-3-(2-methoxy-4-methylmercaptobenzyl)-8-octanoyloxy-10b-(3-butenyl)-benz[f]isoquinoline,

1,2,3,4,4a,5,6,10b-octahydro-3-[2-(4-aminophenyl)ethyl]-9-ethoxy-10b-methylbenz[f]isoquinoline,

1,2,3,4,4a,5,6,10b-octahydro-3-[(2-furyl)-methyl]-10b-allylbenz[f]isoquinolin-9-ol,

1,2,3,4,4a,5,6,10b-octahydro-4-ethyl-10b-methylbenz[f]isoquinolin-7-ol maleate,

1,2,3,4,4a,5,6,10b-octahydro-3-cyclobutylmethyl-9-propoxy-10b-(2-propenyl)benz[f]isoquinoline benzoate,

1,2,3,4,4a,5,6,10b-octahydro-4-propyl-10b-n-propylbenz[f]isoquinolin-8-ol propionate,

1,2,3,4,4a,5,6,10b-octahydro-3-benzyl-9-methoxy-10b-ethylbenz[f]isoquinoline lactate,

1,2,3,4,4a,5,6,10b-octahydro-3-(2-phenylethyl)-10-isopropoxy-10b-propylbenz[f]isoquinoline,

1,2,3,4,4a,5,6,10b-octahydro-10b-methylbenz[f]isoquinolin-9-ol hydroiodide,

1,2,3,4,4a,5,6,10b-octahydro-3-cyclohexylmethyl-4-ethyl-9-lauroyloxy-10b-propylbenz[f]isoquinoline,

1,2,3,4,4a,5,6,10b-octahydro-3-octyl-4-heptyl-8-(4-butyloctanoyloxy)-10b-(5-hexenyl)benz[f]isoquinoline,

1,2,3,4,4a,5,6,10b-octahydro-3-cyclopentylmethyl-10-butanoyloxy-10b-n-butylbenz[f]isoquinoline,

1,2,3,4,4a,5,6,10b-octahydro-10b-propylbenz-[f]isoquinolin-9-ol,

1,2,3,4,4a,5,6,10b-octahydro-3,4-dimethyl-10b-ethylbenz[f]isoquinolin-8-ol toluenesulfonate,

1,2,3,4,4a,5,6,10b-octahydro-4-methyl-9-methoxy-10b-n-butylbenz[f]isoquinoline,

1,2,3,4,4a,5,6,10b-octahydro-3,10b-diethyl-7-isopropoxybenz[f]isoquinoline, and

1,2,3,4,4a,5,6,10b-octahydro-4,10b-dimethylbenz[f]isoquinolin-9-ol.

The compounds of this invention are valuable psychotropic and analgesic agents for treating animals suffering from pain. The analgesic potencies of the compounds of Formula I are comparable to that of meperidine as demonstrated, for instance, in the standard mouse writhing assay. The compounds are not "morphine-like" when tested in mice, and have demonstrated narcotic antagonist-like properties.

The analgesic activity of a number of compounds provided by this invention has been determined in the standard rat-tail jerk assay and the mouse writhing assay. Writhing, characterized by contraction of the abdominal musculature, extension of the hindlegs, and rotation of the trunk, was induced in Cox standard strain albino male mice. The mice, weighing 18-24 grams, were fasted overnight and given the test compound by gavage in an acacia suspension (5%) sixty minutes before writhing was induced by the intraperitoneal administration of acetic acid (0.60 percent). Each treatment group consisted of 5 mice. The total number of writhes for the treatment group was determined during a 10-minute observation starting 5 minutes

after acetic acid administration. Control groups had a total of 40-60 writhes per observation period. Table I shows typical test results obtained with the claimed compounds. The results in the mouse writhing assay are presented as the effective dose in mg./kg. of the tested compound required to inhibit induced writhing in the test animals by fifty percent ($ED_{50}$). Results are presented in Column II for both subcutaneous (s.c.) and oral (p.o.) administration of the test compound as compared to the reference compound meperidine.

Table I

| Column I Compound of Example No. | Column II Mouse Writhing $ED_{50}$ | |
|---|---|---|
| | s.c. | p.o. |
| 1 | 10 | 15 |
| 2 | 10 | 11 |
| 3 | 2.3 | 7.2 |
| 4 | 4.5 | 13 |
| 5 | 62 | 80 |
| 6 | >80 | >80 |
| 7 | 80 | >80 |
| 8 | 15 | >80 |
| 9 | 32 | >80 |
| 10 | 5.2 | 38.2 |
| 11 | 62 | >80 |
| 12 | 12 | 45 |
| 13 | 34 | 73 |
| 14 | 78 | >80 |
| Meperidine | 2.8 | 21 |

A further aspect of this invention provides for pharmaceutical formulations containing the compounds having the above formula. Such formulations are useful in the treatment of pain in warm-blooded animals. The formulations contemplated comprise an analgesically-effective dose of a compound of the invention in combination with any of a number of pharmaceutical diluents, excipients and carriers. Typical diluents commonly used in such formulations may include lactose, sucrose, starch, micro-crystalline cellulose, calcium sulfate, or sodium benzoate. Typical formulations will contain from about 1 to about 30 percent by weight of active ingredient. The formulations can be compressed into tablets or encapsulated in gelatin capsules for convenient oral administration. Alternatively, the formulations can be dissolved in sterile water or saline and placed in a suitable vial for convenient intravenous or intramuscular administration.

The compounds of the invention have demonstrated valuable analgesic activity, and therefore are useful in the treatment of pain. The invention thus further provides a method for imparting analgesia in an animal which comprises administering an analgesically-effective dose of a compound of the invention. Typical doses commonly administered will range from about 0.5 to about 150 mg. per kg. of animal body weight. A preferred dose will be from about 1.0 to about 50 mg./kg. The method of treatment can be accomplished by administering an analgesic compound of the invention via the oral or parenteral routes.

Preferred routes of administration include the oral and intramuscular routes. Subcutaneous administration of the suitably formulated active compounds can also be utilized when desired. As an illustration of the contemplated method of treatment, a formulation comprising about 50 mg. of 1,2,3,4,4a,5,6,10b-octahydro-9-methoxy-10b-n-propylbenz[f]isoquinoline hydrochloride dissolved in about 1 ml. of saline is administered from 1 to 4 times daily to a subject suffering from pain and in need of analgesic treatment.

To more fully illustrate the various aspects of the invention, the following non-limiting examples are provided.

## Preparation 1

1-hydroxy-1-methyl-7-methoxy-1,2,3,4-tetrahydronaphthalene

A solution of 208.8 g. (1.2 moles) of 7-methoxy-1-tetralone in 1800 ml. of anhydrous ether was added dropwise to a solution of 178.8 g. (1.5 moles) of methyl magnesium bromide in 500 ml. of anhydrous ether. After all of the solution was added, the reaction was heated to reflux for 30 minutes, and then quenched with 250 ml. of a saturated ammonium chloride solution. The resulting layers were separated and the organic layer was washed with water, then with saturated sodium chloride solution, and dried over potassium carbonate. The solution was evaporated to dryness, yielding 198.0 g. of a tan solid. Recrystallization from hexane yielded 189.4 g. of the title product as a tan solid, m.p. about 75-77°C.

Analysis: $C_{12}H_{16}O_2$;

Calc: C, 74.97; H, 8.39;

Found: C, 74.68; H, 8.12.

Preparation 2

1-methyl-7-methoxy-3,4-dihydronaphthalene

A solution of 367.2 g. (1.9 moles) of 1-hydroxy-1-methyl-7-methoxy-1,2,3,4-tetrahydronaphthalene and 4.0 g. of p-toluenesulfonic acid in 4 liters of toluene was heated to reflux overnight with water being collected by means of a Dean Stark trap. The reaction mixture was then washed several times with water followed by a saturated sodium chloride solution wash. The organic solution was dried over potassium carbonate and evaporated to dryness yielding 345.8 g. of a brown liquid. Vacuum distillation of the liquid at 84-86°C. and 0.1 mm. of Hg yielded 310.1 g. of the title product as a colorless liquid.

Analysis: $C_{12}H_{14}O$;

Calc: C, 82.72; H, 8.10;

Found: C, 83.02; H, 7.98.

Preparation 3

1,2,3,4,5,6-hexahydro-3-methyl-9-methoxybenz[f]isoquinoline

A solution of 141.1 g. (0.81 moles) of 1-methyl-7-methoxy-3,4-dihydronaphthalene and 263.0 g. of a 37% solution of formaldehyde (3.24 moles) in 700 ml. of acetic acid was heated to 65-70°C. for 30 minutes with stirring. While maintaining the temperature below 70°C., 125.8 g. (1.86 moles) of methylamine hydrochloride were added and the mixture was stirred at 70°C. for 3 hours. The solution was cooled to room temperature, diluted with 1 liter of ice-water, and washed with 3 x 500 ml. of ether. The aqueous layer was made basic with about 500 ml. of 50% sodium hydroxide, and was then extracted with ether. The organic layer was separated and dried over potassium carbonate and evaporated to dryness yielding 163.8 g. of a yellow oil. Vacuum distillation of the oil at 0.01 mm. of Hg between 150 and 205°C. gave 96.2 g. of the title product as a yellow oil. The hydrochloride salt was made using etherial hydrogen chloride. Upon evaporation of the solvent and recrystallization from ether/ethanol, the title product hydrochloride was obtained, m.p. about 229-232°C.

Analysis: $C_{15}H_{19}NO{\cdot}HCl$;

Calc: C, 67.99; H, 7.59; N, 5.27;

Found: C, 67.99; H, 7.39; N, 5.07.

## Preparation 4

1,2,3,5,6,10b-hexahydro-3,10b-dimethyl-9-methoxybenz[f]isoquinoline

A 1.6M solution of n-butyllithium in hexane (118.8 ml., 0.190 moles) was slowly added to a solution of 29.2 g. (0.127 moles) of 1,2,3,4,5,6-hexahydro-3-methyl-9-methoxybenz[f]isoquinoline in 500 ml. of dry tetrahydrofuran at -5°C. under a nitrogen atmosphere. The resulting solution was slowly added to a solution of 24.1 g. (0.254 moles) of methyl bromide which had

previously been condensed into 400 ml. of ether at -75°C. The temperature during the addition was maintained below -60°C. for the 1 hour reaction time. The reaction mixture was then diluted with 500 ml. of ether and 500 ml. of saturated sodium chloride solution, and allowed to warm to room temperature. The mixture was extracted several times with ether and the combined organic layers were washed with water. The organic solution was dried over potassium carbonate and evaporated to dryness yielding 23.4 g. of a yellow-brown oil. Vacuum distillation of the oil at 0.03 mm. of Hg between 117 and 135°C. gave 19.2 g. (62% yield) of the title product as a yellow oil. The perchlorate salt was formed and dissolved in ethyl acetate/ethanol. Upon cooling, 16.2 g. of the perchlorate salt as a gray solid were collected by filtration, m.p. about 172-174°C.

Analysis: $C_{16}H_{21}NO \cdot HClO_4$;

Calc: C, 56.06; H, 6.18; N, 4.09;

Found: C, 56.30; H, 6.42; N, 3.84.

## Preparation 5

cis-1,2,3,5,6,10b-hexahydro-3-methyl-9-methoxy-10b-propylbenz[f]isoquinoline

Following the procedure of preparation 4, 122.0 ml. of 1.6M n-butyllithium in hexane were added to 30.0 g. of 1,2,3,4,5,6-hexahydro-3-methyl-9-methoxybenz[f]isoquinoline in 540 ml. of dry tetrahydrofuran at -5°C. This solution was then added to a solution of 32.0 g. of propyl bromide in 1 liter of anhydrous ethyl ether at -60°C. Upon working up the reaction, 34.4 g.

of a brown oil were obtained. Vacuum distillation at 0.05 mm. of Hg and 124-151°C. yielded 28.9 g. of the title product as a yellow oil. The perchlorate salt was formed and crystallized from ethyl acetate/ethanol yielding 22.1 g. of a tan solid, m.p. about 166-168°C.

Analysis: $C_{18}H_{25}NO \cdot HClO_4$;

Calc: C, 58.14; H, 7.05; N, 3.77;

Found: C, 57.89; H, 6.88; N, 3.55.

## Example 1

cis-1,2,3,4,4a,5,6,10b-octahydro-3,10b-dimethyl-9-methoxybenz[f]isoquinoline hydrochloride

To a solution of 3.0 g. (8.7 mmoles) of cis-1,2,3,5,6,10b-hexahydro-9-methoxy-3,10b-dimethyl-benz[f]isoquinoline perchlorate in 150 ml. of ethanol was added 3.0 g. (79 mmoles) of sodium borohydride. The reaction mixture was stirred at room temperature for 1 hour and was then diluted with water. The solution was extracted with ether, and the organic layer was first washed with water and then dried over potassium carbonate. On evaporation of the solvent, 2.5 g. of a yellow oil were obtained. The hydrochloride salt was formed in etherial hydrogen chloride yielding 3.0 g. of a white solid which was crystallized from ethyl acetate/ethanol. The first crop yielded 1.6 g. of the title product and the second crop yielded 0.13 g. of the title product, both crops having a m.p. of about 239-240°C. The two crops were combined for analysis.

Analysis: $C_{16}H_{23}NO \cdot HCl$;

Calc: C, 68.19; H, 8.58; N, 4.97;

Found: C, 68.00; H, 8.43, N, 4.76.

Example 2

cis-1,2,3,4,4a,5,6,10b-octahydro-3-methyl-9-methoxy-10b-propylbenz[f]isoquinoline hydrochloride

Following the procedure of Example 1, 3.9 g. of cis-1,2,3,5,6,10b-hexahydro-3-methyl-9-methoxy-10b-propylbenz[f]isoquinoline perchlorate were treated with 3.9 g. of sodium borohydride to give 2.9 g. of a yellow oil. Preparation and crystallization of the hydrochloride salt yielded 1.33 g. of the title product as a white solid, m.p. about 260-261°C.

Analysis: $C_{18}H_{27}NO \cdot HCl$;

Calc: C, 69.77; H, 9.11; N, 4.52;

Found: C, 69.50; H, 8.90; N, 4.59.

Example 3

cis-1,2,3,4,4a,5,6,10b-octahydro-3,10b-dimethylbenz[f]isoquinolin-9-ol

A solution of 5.1 g. (20.8 mmoles) of cis-1,2,3,4,4a,5,6,10b-octahydro-3,10b-dimethyl-9-methoxybenz[f]isoquinoline in 50 ml. of acetic acid and 50 ml. of 48% hydrobromic acid was heated to reflux overnight. The reaction mixture was diluted with 500 ml. of water and neutralized with 50% sodium hydroxide. The solution was brought to pH 10.2 with 1N sodium hydroxide and extracted into 400 ml. of 3:1 butanol/toluene. The

organic layer was dried over potassium carbonate and evaporated to dryness yielding 4.8 g. of a yellow solid. Crystallization of the solid from ethyl acetate/ ethanol gave two crops of the title product as a tan solid, total yield of 2.69 g., m.p. about 218-220°C.

Analysis: $C_{15}H_{21}NO$;

Calc: C, 77.88; H, 9.15; N, 6.05;

Found: C, 78.16; H, 9.17; N, 6.25.

## Example 4

cis-1,2,3,4,4a,5,6,10b-octahydro-3-methyl-10b-propylbenz[f]isoquinolin-9-ol hydrochloride

Following the procedure of Example 3, 3.1 g. of cis-1,2,3,4,4a,5,6,10b-octahydro-3-methyl-9-methoxy-10b-propylbenz[f]isoquinoline hydrochloride were heated with 50 ml. of acetic acid and 50 ml. of 48% hydrobromic acid. The free base of the title product was obtained as white crystals, m.p. about 195-196°C., which was converted to the title hydrochloride salt. Crystallization from ethanol/Skelly B yielded 1.2 g. of the title product, m.p. about 222-224°C.

Analysis: $C_{17}H_{25}NO \cdot HCl$;

Calc: C, 69.01; H, 8.86; N, 4.74;

Found: C, 68.93; H, 8.75; N, 4.61.

Example 5

cis-1,2,3,4,4a,5,6,10b-octahydro-9-methoxy-10b-methylbenz[f]isoquinoline hydrochloride

Eleven millimoles (2.7 g.) of cis-1,2,3,4,4a,-5,6,10b-octahydro-3,10b-dimethyl-9-methoxybenz[f]isoquinoline were dissolved in 40 ml. of methylene chloride and a solution of 1.7 g. (11.0 mmoles) of phenyl chloroformate in 10 ml. of methylene chloride was slowly added. After refluxing the reaction mixture for 4 hours, the mixture was evaporated to dryness and the resulting oil was dissolved in 50 ml. of 5% sodium hydroxide. The product was extracted into ether and the ether was washed with water, 10% hydrochloric acid, and water again. After drying over potassium carbonate, the solution was evaporated to dryness yielding 3.3 g. of a colorless oil. The oil was dissolved in 100 ml. of ethanol and 27 ml. of 50% aqueous potassium hydroxide. The solution was refluxed for 60 hours and then cooled to room temperature. The ethanol was evaporated and the solution was made acidic with concentrated hydrochloric acid. The solution was washed with ether and then made basic with 50% sodium hydroxide. The basic solution was extracted with ether. The ether solution was dried over potassium carbonate and was evaporated to dryness yielding 1.7 g. of a yellow oil. The oil was converted into the hydrochloride salt and crystallized from ethyl acetate/ethanol giving 1.5 g. of the title product as a white solid, m.p. about 219-220°C.

Analysis: $C_{15}H_{21}NO \cdot HCl$;

Calc: C, 67.28; H, 8.28; N, 5.23;
Found: C, 67.03; H, 8.41; N, 5.09.

Example 6

cis-1,2,3,4,4a,5,6,10b-octahydro-9-methoxy-10b-propylbenz[f]isoquinoline hydrochloride

Following the procedure of Example 5, 5.0 g. (18 mmoles) of cis-1,2,3,4,4a,5,6,10b-octahydro-3-methyl-9-methoxy-10b-propylbenz[f]isoquinoline were transformed into 1.24 g. of the title product, m.p. about 198-200°C.

Analysis: $C_{17}H_{25}NO \cdot HCl$;

Calc: C, 69.02; H, 8.86; N, 4.73;
Found: C, 68.82; H, 8.81; N, 4.53.

Example 7

cis-1,2,3,4,4a,5,6,10b-octahydro-10b-methylbenz[f]isoquinolin-9-ol

Following the procedure of Example 3, 8.8 g. (38 mmoles) of cis-1,2,3,4,4a,5,6,10b-octahydro-9-methoxy-10b-methylbenz[f]isoquinoline were refluxed overnight in 100 ml. of acetic acid and 100 ml. of 48% hydrobromic acid. After work-up and crystallization from ethyl acetate/ethanol, 2.64 g. of the title product were obtained as a tan solid, m.p. about 253-255°C.

Analysis: $C_{14}H_{19}NO$;

Calc: C, 77.38; H, 8.81; N, 6.45;
Found: C, 77.08; H, 8.55; N, 6.19.

## Example 8

cis-1,2,3,4,4a,5,6,10b-octahydro-3-(2-propenyl)-10b-methylbenz[f]isoquinolin-9-ol

A solution of 2.0 g. (9 mmoles) of cis-1,2,3,4,4a,5,6,10b-octahydro-10b-methylbenz[f]isoquinolin-9-ol, 1.2 g. (10 mmoles) of allyl bromide, and 1.9 g. (22 mmoles) of sodium bicarbonate in 50 ml. of dimethylformamide was refluxed for 1 hour, cooled and poured into 500 ml. of water. The solution was extracted with 1 liter of ether and the layers were separated. The ether layer was washed several times with water, dried over potassium carbonate, and evaporated to dryness. The residue was crystallized from ethyl acetate giving 1.07 g. of the title product as yellow-white crystals, m.p. about 164-165°C.

Analysis: $C_{17}H_{23}NO$;

Calc: C, 79.33; H, 9.01; N, 5.44;
Found: C, 79.60; H, 9.26; N, 5.23.

## Example 9

cis-1,2,3,4,4a,5,6,10b-octahydro-3-(cyclopropylmethyl)-10b-methylbenz[f]isoquinolin-9-ol

A solution of 2.0 g. (10 mmoles) of cis-1,2,3,4,4a,5,6,10b-octahydro-10b-methylbenz[f]isoquinolin-9-ol, 2.5 g. (25 mmoles) of triethylamine, and 2.1 g. (20 mmoles) of cyclopropanecarboxylic acid chloride in 45 ml. of dimethylformamide was heated to 70°C. for 90 minutes. After cooling, the reaction mixture was poured into 300 ml. of water and extracted

with 500 ml. of ether. The ether layer was dried over potassium carbonate and evaporated to dryness yielding a tan foam. The foam was dissolved in 75 ml. of dry tetrahydrofuran and added dropwise to 1.0 g. of lithium aluminum hydride in 100 ml. of tetrahydrofuran. The mixture was refluxed for 4 hours and then quenched with 10 ml. of ethyl acetate. Inorganic salts were precipitated by the addition of a saturated ammonium chloride solution. The organic layer was decanted and the salts were washed with tetrahydrofuran. The combined tetrahydrofuran layer and washings were evaporated to dryness and then taken up into ether. The ether layer was washed with water, dried over potassium carbonate, and evaporated to dryness. The residue was dissolved in 500 ml. of 1N hydrochloric acid and washed with ether. The acid layer was made basic with 50% sodium hydroxide and extracted into ether. The ether layer was washed with water, dried over potassium carbonate, and evaporated to dryness yielding 0.5 g. of a white solid. Recrystallization of the solid from ethyl acetate yielded 0.26 g. of the title product as a white solid, m.p. about 202-204°C.

Analysis: $C_{18}H_{25}NO$;

Calc: C, 79.36; H, 9.62; N, 5.14;

Found: C, 79.32; H, 9.49; N, 4.98.

Example 10

cis-1,2,3,4,4a,5,6,10b-octahydro-3-(2-phenylethyl)-10b-methylbenz[f]isoquinolin-9-ol

Following the procedure of Example 9, 2.75 g. (13 mmoles) of cis-1,2,3,4,4a,5,6,10b-octahydro-10b-methylbenz[f]isoquinolin-9-ol were converted to the title product by first heating with 3.24 g. (32 mmoles) of triethylamine and 4.94 g. (32 mmoles) of phenylacetyl chloride in 90 ml. of dimethylformamide. The resulting oil was dissolved in 120 ml. of tetrahydrofuran and added to 1.8 g. of lithium aluminum hydride in 140 ml. of tetrahydrofuran. Crystallization of the resulting product from ethyl acetate/ethanol gave 0.56 g. of the title product as a white solid, m.p. about 220-222°C.

Analysis: $C_{22}H_{27}NO$;

Calc: C, 82.20; H, 8.47; N, 4.36;
Found: C, 81.98; H, 8.70; N, 4.69.

Example 11

cis-1,2,3,4,4a,5,6,10b-octahydro-10b-propylbenz[f]isoquinolin-9-ol

Following the procedure of Example 7, 3.5 g. (13 mmoles) of cis-1,2,3,4,4a,5,6,10b-octahydro-9-methoxy-10b-propylbenz[f]isoquinoline were treated with 30 ml. of acetic acid and 30 ml. of 48% hydrobromic acid to give 1.4 g. of the title product (crystallized from ethyl acetate/ethanol) as a white solid, m.p. about 202-203°C.

Analysis: $C_{16}H_{23}NO$;

Calc: C, 78.32; H, 9.45; N, 5.71;

Found: C, 78.04; H, 9.24; N, 5.61.

Example 12

cis-1,2,3,4,4a,5,6,10b-octahydro-3-(2-propenyl)-10b-propylbenz[f]isoquinolin-9-ol

Following the procedure of Example 8, 2.0 g. (8 mmoles) of cis-1,2,3,4,4a,5,6,10b-octahydro-10b-propylbenz[f]isoquinolin-9-ol were transformed into 1.1 g. of the title product as a white solid, m.p. about 148-149°C.

Analysis: $C_{19}H_{27}NO$;

Calc: C, 79.95; H, 9.54; N, 4.91;

Found: C, 80.16; H, 9.80; N, 4.70.

Example 13

cis-1,2,3,4,4a,5,6,10b-octahydro-3-cyclopropylmethyl-10b-propylbenz[f]isoquinolin-9-ol

Following the procedure of Example 9, 2.0 g. (10 mmoles) of cis-1,2,3,4,4a,5,6,10b-octahydro-10b-propylbenz[f]isoquinolin-9-ol were transformed into 2.1 g. of the title product as a white solid, m.p. about 182-183°C.

Analysis: $C_{20}H_{29}NO$;

Calc: C, 80.22; H, 9.76; N, 4.68;

Found: C, 79.97; H, 9.51; N, 4.61.

Example 14

cis-1,2,3,4,4a,5,6,10b-octahydro-3-(2-phenylethyl)-10b-propylbenz[f]isoquinolin-9-ol

Following the procedure of Example 10, 4.0 g. (15 mmoles) of cis-1,2,3,4,4a,5,6,10b-octahydro-10b-propylbenz[f]isoquinolin-9-ol were converted into 0.3 g. of the title product, m.p. about 214-216°C.

Analysis: $C_{24}H_{31}NO$;

Calc: C, 82.47; H, 8.94; N, 4.01;

Found: C, 82.21; H, 9.11; N, 3.91.

## Example 15

cis-1,2,3,4,4a,5,6,10b-octahydro-3-(2-methyl-2-propenyl)-10b-methylbenz[f]isoquinolin-9-ol

Following the procedure of Example 8, 1.18 g. (5 mmoles) of cis-1,2,3,4,4a,5,6,10b-octahydro-10b-methylbenz[f]isoquinolin-9-ol, 0.62 g. of sodium bicarbonate, and 0.60 g. of 3-chloro-2-methylpropene in 25 ml. of dimethylformamide were heated to 65-70°C. for one hour and worked up as before to give 320 mg. of the title product, m.p. about 138.5-140.5°C.

Analysis: $C_{18}H_{25}NO$;

Calc: C, 79.66; H, 9.29; N, 5.16;

Found: C, 79.43; H, 9.05; N, 4.96.

## Example 16

cis-1,2,3,4,4a,5,6,10b-octahydro-3-(3-butenyl)-10b-methylbenz[f]isoquinolin-9-ol

Following the procedure of Example 8, 0.63 g. of cis-1,2,3,4,4a,5,6,10b-octahydro-10b-methylbenz[f]-isoquinolin-9-ol, 0.35 g. of sodium bicarbonate, and 0.46 g. of 4-bromo-1-butene in 25 ml. of dimethylformamide were heated to 75-85°C for one hour and worked up as before. Crystallization from 25 ml. of

n-hexane and 12 ml. of ethyl acetate gave 496 mg. of the title product, m.p. about 166.5-168°C.

Analysis: $C_{18}H_{25}NO$;
Calc: C, 79.66; H, 9.29; N, 5.16;
Found: C, 79.42; H, 9.23; N, 4.96.

Example 17

cis-1,2,3,4,4a,5,6,10b-octahydro-3-propyl-10b-methylbenz[f]isoquinolin-9-ol

Following the procedure of Example 8, 0.63 g. of cis-1,2,3,4,4a,5,6,10b-octahydro-10b-methylbenz[f]isoquinolin-9-ol, 0.35 g. of sodium bicarbonate, and 0.42 g. of propyl bromide in 25 ml. of dimethylformamide were heated to 65-70°C for one hour. After working up the reaction and crystallization from 25 ml. of n-hexane and 15 ml. of ethyl acetate, 269 mg. of the title product were obtained, m.p. about 172-174°C.

Analysis: $C_{17}H_{25}NO$;
Calc: C, 78.72; H, 9.71; N, 5.40;
Found: C, 78.52; H, 9.62; N, 5.25.

CLAIMS

1. A compound of Formula (I),

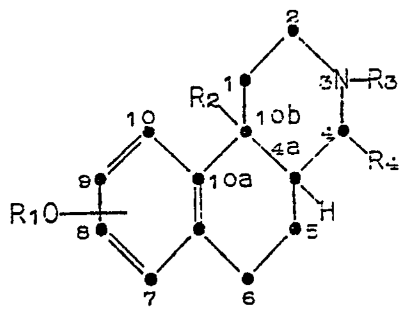 I

or a pharmaceutically-acceptable salt thereof, in which

$R_1$ is hydrogen, $C_1$-$C_4$ alkyl, or $C_1$-$C_{12}$ alkanoyl;

$R_2$ is $C_1$-$C_4$ alkyl, $C_2$-$C_6$ alkenyl, or

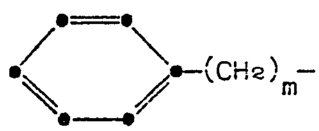

in which m is 1, 2, or 3;

$R_3$ is hydrogen, $C_1$-$C_8$ alkyl, $C_2$-$C_6$ alkenylmethyl, ($C_3$-$C_8$ cycloalkyl)methyl,

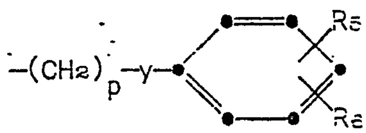, or

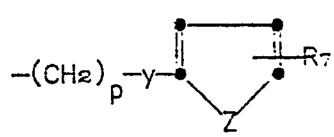,

in which p is 0, 1, or 2;

Y is $-\overset{O}{\overset{\|}{C}}-$, $-\overset{OH}{\overset{|}{CH}}-$, $-CH_2-$, -O-, -S-, or -NH-, providing that when Y is -O-, -S-, or -NH-, p may only be 2, and providing that when Y is $-\overset{OH}{\overset{|}{CH}}-$ , p may not be 0;

Z is O or S;

$R_5$ is $C_1$-$C_4$ alkylthio, nitro, amino, trifluoromethyl, hydroxy, hydrogen, $C_1$-$C_4$ alkyl, $C_1$-$C_4$ alkoxy, or halo;

$R_6$ is hydrogen, $C_1$-$C_4$ alkyl, $C_1$-$C_4$ alkoxy, or halo;

$R_7$ is hydrogen or methyl; and

$R_4$ is $C_1$-$C_8$ alkyl or hydrogen.

2. A compound as claimed in claim 1 in which $R_1$ is hydrogen or $C_1$-$C_4$ alkyl, $R_2$ is $C_1$-$C_4$ alkyl, $R_3$ is hydrogen, $C_1$-$C_4$ alkyl, $C_2$-$C_6$ alkenylmethyl, ($C_3$-$C_8$ cycloalkyl)methyl or

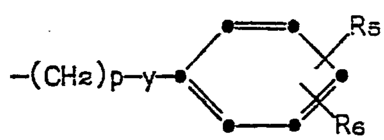

in which p, y, $R_5$ and $R_6$ are as defined in claim 1, or $R_4$ is hydrogen, or a pharmaceutically-acceptable salt thereof.

3. A compound as claimed in claim 1 or 2 in which $R_1$ is hydrogen or methyl, $R_2$ is methyl or n-propyl, $R_3$ is hydrogen, methyl, 2-propenyl, cyclopropylmethyl or phenylethyl, or $R_4$ is hydrogen, or a pharmaceutically-acceptable salt thereof.

4. cis-1,2,3,4,4a,5,6,10b-Octahydro-3,10b-dimethylbenz[f]isoquinolin-9-ol, or a pharmaceutically-acceptable salt thereof.

5. cis-1,2,3,4,5a,5,6,10b-Octahydro-3-methyl-10b-propylbenz[f]isoquinolin-9-ol, or a pharmaceutically-acceptable salt thereof.

6. A process for preparing a compound of Formula (I), as claimed in any one of claims 1 to 5, or a pharmaceutically-acceptable salt thereof, which comprises:

(a) Reducing a compound of Formula (II),

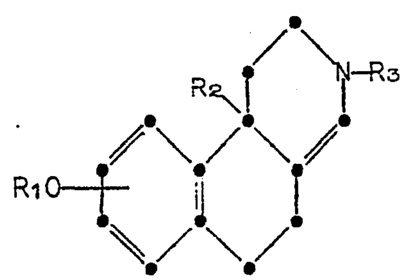

(II)

to form a compound of Formula (I) in which $R_4$ is hydrogen; or,

(b) Reacting a compound of Formula (III),

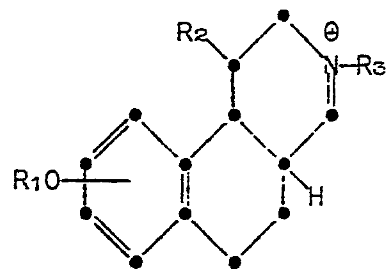

(III)

in which $R_1$ is $C_1$-$C_4$ alkyl, with an organometallic reagent to form a compound of Formula (I), in which $R_4$ is $C_1$-$C_8$ alkyl; or,

(c) Reducing a compound of Formula III to form a compound of Formula I in which $R_4$ is hydrogen; or

(d) Hydrolysis or reduction of a compound of Formula (I) in which $R_1$ is $C_1$-$C_4$ alkyl or $C_1$-$C_{12}$ alkanoyl, to form a compound in which $R_1$ is hydrogen; or,

(e) Acylation or alkylation of a compound of Formula (I) in which $R_1$ is H, to form a compound of Formula (I) in which $R_1$ is alkanoyl or alkyl ; or

(f) Treatment of a compound of Formula (I), in which $R_3$ is $-\underset{O}{\overset{\|}{C}}-OW$ where W is $C_1$-$C_4$ alkyl, benzyl or phenyl, with a base to form a compound of Formula (I) in which $R_3$ is hydrogen; or,

(g) Reacting a compound of Formula (I) in which $R_3$ is hydrogen, with an alkyl halide, or

acyl halide followed by reduction, to form a compound of Formula (I) in which $R_3$ is other than hydrogen; and,

(h) if desired, salifying a product of any of reactions (a)-(g).

7. A compound of Formula (I), or a pharmaceutically-acceptable salt thereof, as claimed in any of claims 1 to 5, for use as an analgesic or psychotropic agent in the chemotherapy of warm-blooded animals.

8. A pharmaceutical formulation which comprises as an active ingredient, a compound of Formula (I), or a pharmaceutically-acceptable salt thereof, as claimed in any one of claims 1 to 5, associated with one or more physiologically-acceptable carriers or vehicles therefor.